# EUROPÄISCHE PATENTSCHRIFT

(11) **EP 3 402 386 B1**
(45) Veröffentlichungstag und Bekanntmachung des Hinweises auf die Patenterteilung: **16.07.2025**
(21) Anmeldenummer: 16700558.6
(22) Anmeldetag: 12.01.2016
(51) Int. Cl.: A61B 3/032, A61B 3/00, A61B 3/06

(54) **VERFAHREN ZUR SEHZEICHENDARSTELLUNG UND SEHZEICHENGERÄT**
METHOD FOR REPRESENTING OPTOTYPES AND OPTOTYPE PRESENTING APPARATUS
PROCÉDÉ POUR REPRÉSENTER DES OPTOTYPES ET APPAREIL À OPTOTYPES

(43) Veröffentlichungstag der Anmeldung: 21.11.2018
(73) Patentinhaber: Deutsche Augenoptik GmbH, 75417 Mühlacker (DE)
(72) Erfinder: STELZER, Lars-Erik, 75233 Tiefenbronn (DE)
(74) Vertreter: DREISS Patentanwälte PartG mbB
(86) Internationale Anmeldenummer: PCT/EP2016/050481
(87) Internationale Veröffentlichungsnummer: WO 2017/121466

(56) Entgegenhaltungen:
- WO-A1-2015/028721
- CN-U- 201 477 825
- DE-U1- 202015 004 457
- US-A- 5 121 981
- US-A1- 2007 273 833
- US-A1- 2011 051 019

## Beschreibung

Die Erfindung betrifft ein Verfahren zur Bestimmung einer bei Nacht geeigneten Augenkorrektur unter Berücksichtigung dämmerungs- und nachtbedingter Effekte bei der Refraktion an einer Testperson sowie ein zugehöriges Sehzeichengerät mit einer Anzeige und einem Rahmen.

Bei der menschlichen Wahrnehmung des Sehens unterscheidet man zwischen der photopischen Wahrnehmung, dem sogenannten Tagsehen, der mesopischen Wahrnehmung, dem sogenannten Dämmerungssehen und der skotopischen Wahrnehmung, dem sogenannten Nachtsehen.

In der Optometrie und Augenheilkunde wird die Refraktion üblicherweise bei Tageslichtverhältnissen bestimmt. Unter Refraktion versteht man den Brechwert der optischen Korrektur, mit der zusammen ein bestimmtes Auge ohne Akkomodation ein scharfes Bild eines in unendlicher Entfernung befindlichen Objekts erzeugt.

Brillenträger, deren Refraktion bei Tageslichtverhältnissen ermittelt wurde, klagen jedoch bei schlechten Lichtverhältnissen oftmals über Sehschwierigkeiten, bspw. beim Autofahren in der Dämmerung (mesopisches Sehen) oder bei Nacht (skotopisches Sehen).

Die Brillenträger klagen dabei oftmals darüber, dass entgegenkommende Fahrzeuge sehr stark blenden. Eine Ursache hierfür kann darin liegen, dass die Korrektur der Brillengläser nicht exakt an die Augen des Brillenträgers angepasst ist. Da die Blendung jedoch ein subjektiver Eindruck ist, ist eine Messung bislang schwierig durchzuführen. Eine weitere Ursache hierfür kann in Medientrübungen, d.h. in krankheitsbedingten Trübungen der Augen, wie bspw. dem grauen Star liegen.

Krankheitsbedingte Trübungen lassen sich nur operativ beheben. Ein erhöhter Blendeindruck aufgrund einer nicht exakt an das Auge des Brillenträgers angepassten Brille kann jedoch durch eine neue Brille behoben werden, bei deren Vermessung bereits der subjektive Blendeindruck berücksichtigt ist.

Zur Refraktion von Schielen bzw. Strabismus ist es bekannt, mittels des sogenannten Maddox-Tests eine Testperson zu vermessen, indem man den Brillenträger an ein sogenanntes Maddox-Kreuz mit einer Skala an einer Wand blicken lässt, wobei in der Mitte des Kreuzes eine besonders helle Leuchte angeordnet ist. Ein Auge der Testperson soll dabei auf die zentrale Leuchte schauen, wobei das andere Auge durch einen sogenannten Maddox-Zylinder hindurchsieht, der aus dem Licht der Leuchte eine Brennlinie erzeugt. Zur Feststellung der Abweichung muss die Testperson dann die Lage der Brennlinie im Vergleich zu der Leuchte durch die Skala am Maddox-Kreuz beurteilen, wobei verschiedene Abweichungen, horizontal und vertikal, unterschieden werden.

Ein Problem des bekannten Maddox-Testverfahrens ist, dass eine Testperson durch die zentrale Anordnung der Leuchte im Auge, das auf die Leuchte blickt, stark geblendet wird. Dies führt bei der Refraktion zu langen Adaptionsphasen von teilweise mehr als 10 Minuten.

Darüber hinaus leiden viele Menschen unter Nachtkurzsichtigkeit, der sogenannten Nachtmyopie. Als Nachtblind werden dabei Menschen bezeichnet, bei denen der Unterschied zum Tag mehr als -0,5 Dioptrien beträgt.

Eine Ursache dafür ist, dass ein menschliches Auge aufgrund der bei Nacht vergrößerten Pupille weniger Tiefenschärfe bereitstellen kann.

Fehlt dem menschlichen Auge darüber hinaus ein Akkomodationsreiz, so schaltet das Auge auf Entspannung und der Blick fällt ins Unendliche. Bei Dunkelheit und Dämmerung fällt der Blick jedoch in einen Bereich von etwa 1 bis 3 Metern mit relativ großen Schwankungen. Dies wird auch als Kurzsichtigkeit (Myopie) des leeren Raumes bezeichnet. Eine derartige Myopie des leeren Raumes ist mit bekannten Autorefraktionsverfahren nicht feststellbar.

Eine weitere Ursache für Nachtmyopie kann der sogenannte Purkinje-Effekt sein, der das unterschiedliche Helligkeitsempfinden für Farben bei Tag und Nacht bezeichnet. Der Purkinje-Effekt beruht auf der unterschiedlichen spektralen Empfindlichkeit der Sehzellen bei Tag- und Nachtsehen, wobei bei Tageslicht vor allem die Zapfen aktiv sind und in der Nacht vor allem die Stäbchen.

Weil die Stäbchen verstärkt auf Licht mit einer Wellenlänge im Bereich von etwa 500 nm reagieren (blaugrünes Licht), verschiebt sich bei Dämmerungs- und Nachtverhältnissen die Empfindlichkeit der menschlichen Netzhaut in diese Richtung.

Aus der WO 2015/028721 A1 ist ein Verfahren zum Testen und Bestimmen eines Schwellenwerts für psychometrische Tests auf dem Gebiet der Ophthalmologie, der Neuro-Ophthalmologie und der visuellen Neuropsychologie bekannt. In der US 5,121,981 ist ein Sehschärfetestgerät offenbart, das sowohl dazu geeignet ist, die Sehschärfe eines Patienten zu messen, als auch als integraler optischer Verschreibungsrechner fungiert. Aus der DE 20 2015 004 457 U1 ist ein Rahmen für Sehzeichengeräte bekannt, der Lichtquellen besitzt für die Refraktion und/oder Demonstration von Sehleistung in Dämmerung und unter Blendung. Aus der US 2007/0273833 A1 ist ein Verfahren und eine Vorrichtung zum Messen des Netzhautstreulichts im Auge eines Subjekts bekannt. Aus der US 2011/0051019 A1 und der CN 201 477 825 U sind ein steuerbarer Lichtrahmen für ein Display bekannt.

Der Erfindung liegt daher die Aufgabe zugrunde, ein Verfahren zur Bestimmung dämmerungs- und nachtbedingter Effekte bei der Refraktion an einer Testperson sowie ein zugehöriges Sehzeichengerät bereitzustellen, dass die genannten Probleme vermeidet und eine verbesserte Refraktion auch für Dämmerungs- und Nachtverhältnisse ermöglicht.

Diese Aufgabe wird durch ein Verfahren mit den Merkmalen des Anspruchs 1 gelöst.

Die Lichtquellen beleuchten den Abschnitt dabei vorzugsweise derart, dass Licht in Richtung einer auf die Anzeige blickenden Testperson, d.h. vom Rahmen weg ausgesendet wird. Durch die Verwendung der Lichtquellen in verschiedenen Farben wird die chromatische Aberration des Auges genutzt. Jede Lichtfarbe hat eine andere Refraktion. So kann die Refraktion beim mesopischen oder skotopischen Sehen für die benötigten Farben angepasst werden, bspw. für Verkehrssituationen bei Nacht. Menschen mit einer Fehlsichtigkeit nehmen Zerstreuungskreise der verschiedenen Farben (bspw. grün und rot oder blau und rot) in unterschiedlicher Größe wahr, so dass eine Testperson ihren subjektiven Eindruck erfindungsgemäß entsprechend mitteilen kann. Um die Größe der Zerstreuungskreise besser unterscheiden zu können sind die Lichtquellen linienförmig angeordnet. Vorteilhafterweise sind die Lichtquellen dabei in einem Abstand von maximal 10 cm angeordnet. Somit ist es möglich, erfindungsgemäß auch eine bei Nacht geeignete Augenkorrektur zu bestimmen. Vorteilhafterweise wird wenigstens eine rote und wenigstens eine grüne Lichtquelle verwendet. Mit einem derartigen Verfahren können folglich mit Hilfe der Lichtquellen erster Art dämmerungs- und nachtbedingte Effekte bei der Refraktion berücksichtigt werden, so dass bspw. eine für Testpersonen geeignete Nachtfahrbrille bestimmt werden kann.

Erfindungsgemäß wird der Abschnitt mit wenigstens drei am Rahmen angeordneten punktförmigen Lichtquellen erster Art mit Licht von voneinander verschiedener Wellenlänge beleuchtet.

Besonders bevorzugt ist es dabei, wenn der Abschnitt mit farbigen Lichtquellen erster Art, vorzugsweise mit einer roten, einer gelben und einer grünen Lichtquelle oder einer roten, einer grünen und einer blauen Lichtquelle beleuchtet wird.

Eine vorteilhafte Ausgestaltung des Verfahrens sieht vor, dass der Abschnitt mit einer Lichtquelle zweiter Art mit einer Lichtstärke im Bereich von 500 Lumen bis 5000 Lumen verwendet wird. Diese Lichtstärke hat sich als besonders vorteilhaft erwiesen, um die Blendwirkung von bei Dämmerung oder Nacht entgegenkommenden Fahrzeugen zu simulieren.

Eine weitere vorteilhafte Ausgestaltung des Verfahrens sieht vor, dass der Abschnitt mit wenigstens einer dezentralen Lichtquelle dritter Art beleuchtet wird. Vorteilhafterweise umfasst die Lichtquelle dritter Art mehrere linienförmig angeordnete Einzellichtquellen, so dass die Lichtquelle dritter Art ein linienförmiges horizontales und/oder vertikales Licht aussendet. Die Verwendung einer dezentralen Lichtquelle, ist besonders vorteilhaft, da die beim bekannten Maddox-Test auftretende Blendung des auf ein Testkreuz blickenden Auges einer Testperson vermieden werden kann. Als besonders vorteilhaft hat es sich dabei erwiesen, wenn auf der Anzeige eine horizontale oder vertikale Maddox-Testskala dargeboten wird.

Vorteilhafterweise werden als Lichtquellen LEDs verwendet werden. LEDs können aufgrund ihrer geringen Baugröße besonders einfach in einem eine Anzeige umgebenden Rahmen angeordnet werden.

Weiterhin ist es vorteilhaft, wenn das Verfahren in einem Raum ohne Tageslicht und ohne eine weitere künstliche Lichtquelle durchgeführt wird. Dazu kann insbesondere vorgesehen sein, den Raum so weit abdunkeln, dass von einer Testperson keine Farben mehr unterschieden werden können.

Zur Durchführung des Verfahrens wird ein Sehzeichengerät mit einer zentralen Anzeige und mit einem Rahmen mit den Merkmalen des Anspruchs 7 vorgeschlagen. Der Rahmen ist zur Umrahmung der Anzeige ausgelegt und zeichnet sich dadurch aus, dass der Rahmen wenigstens drei punktförmige Lichtquellen erster Art umfasst, die zur Aussendung von Licht von voneinander verschiedener Wellenlänge ausgelegt sind. Vorteilhafterweise ist eine der Lichtquellen zur Aussendung von grünem Licht ausgelegt, wobei eine der Lichtquellen zur Aussendung von rotem Licht ausgelegt ist. Durch die Verwendung der Lichtquellen in verschiedenen Farben wird die chromatische Aberration des Auges genutzt. Jede Lichtfarbe hat eine andere Refraktion. So kann die Refraktion beim mesopischen oder skotopischen Sehen für die benötigten Farben angepasst werden, bspw. für Verkehrssituationen bei Nacht. Menschen mit einer Fehlsichtigkeit nehmen Zerstreuungskreise der verschiedenen Farben (bspw. grün und rot oder blau und rot) in unterschiedlicher Größe wahr, so dass eine Testperson ihren subjektiven Eindruck entsprechend mitteilen kann.

Um die Größe der Zerstreuungskreise besser unterscheiden zu können ist es besonders vorteilhaft, wenn die Lichtquellen erster Art benachbart zueinander, vorzugsweise in einem Abstand von maximal 10 cm, erfindungsgemäß linienförmig angeordnet sind. Bei einem derartigen Abstand der verschiedenfarbigen Lichtquellen erster Art kann eine Testperson ihre subjektiven Eindrücke bei der Refraktion deutlicher mitteilen.

Es hat sich als besonders vorteilhaft erwiesen, wenn der Rahmen erfindungsgemäß wenigstens drei punktförmige Lichtquellen erster Art umfasst, die zur Aussendung von Licht von voneinander verschiedener Wellenlänge ausgelegt sind. Erfindungsgemäß umfassen die drei punktförmigen Lichtquellen erster Art wenigstens eine rote, eine gelbe und eine grüne Lichtquelle oder wenigstens eine rote, eine grüne und eine blaue Lichtquelle.

Weiterhin sieht der Rahmen vor, dass eine Lichtquelle zweiter Art vorgesehen ist, wobei diese Lichtquelle eine Lichtstärke im Bereich von 500 Lumen bis 5000 Lumen aufweist. Diese Lichtstärke hat sich als besonders vorteilhaft erwiesen, um die Blendwirkung von bei Dämmerung oder Nacht entgegenkommenden Fahrzeugen zu simulieren.

Eine weitere vorteilhafte Weiterbildung des Rahmens sieht vor, dass wenigstens eine dezentrale Lichtquelle dritter Art vorgesehen ist. Vorteilhafterweise umfasst die Lichtquelle dritter Art mehrere linienförmig angeordnete Einzellichtquellen, so dass die Lichtquelle dritter Art ein linienförmiges horizontales und/oder vertikales Licht aussendet. Die Verwendung einer dezentralen Lichtquelle dritter Art im Rahmen, die ein linienförmiges Licht aussendet, ist besonders vorteilhaft, da die beim bekannten Maddox-Test auftretende zentrale Blendung des auf ein Testkreuz blickenden Auges vermieden werden kann und die dezentrale Blendung auf eine größere Netzhautfläche verteilt werden kann.

Als besonders vorteilhaft hat es sich dabei erwiesen, wenn die Anzeige ein Display eines 3D-Fernsehers ist.

Weitere Einzelheiten und vorteilhafte Weiterbildungen der Erfindung sind der nachfolgenden Beschreibung zu entnehmen, anhand derer eine Ausführungsform der Erfindung näher beschrieben und erläutert ist.

Es zeigen:

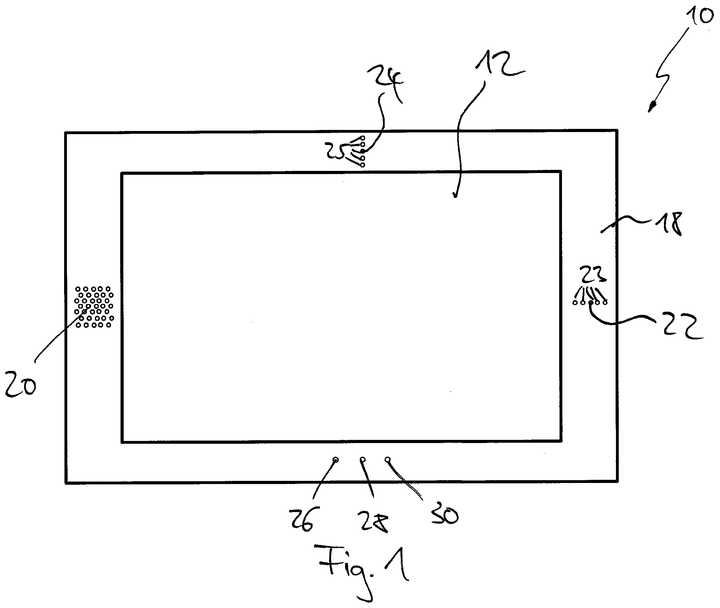

Figur 1 ein erfindungsgemäßes Sehzeichengerät;
Figur 2 das Sehzeichengerät gemäß Figur 1 mit einer Darstellung einer horizontalen Maddox-Linie; und
Figur 3 das Sehzeichengerät gemäß Figur 1 mit einer Darstellung einer vertikalen Maddox-Linie.

In den Figuren ist ein erfindungsgemäßes Sehzeichengerät 10 zur Darbietung von Sehzeichen gezeigt.

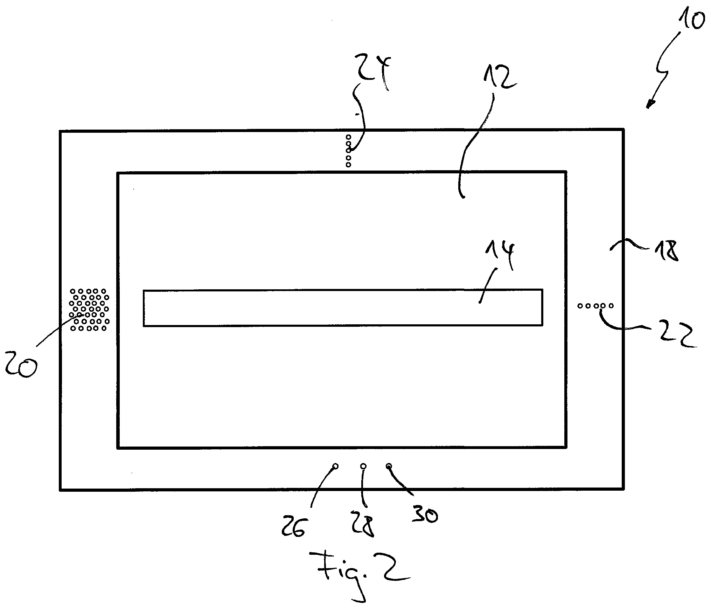

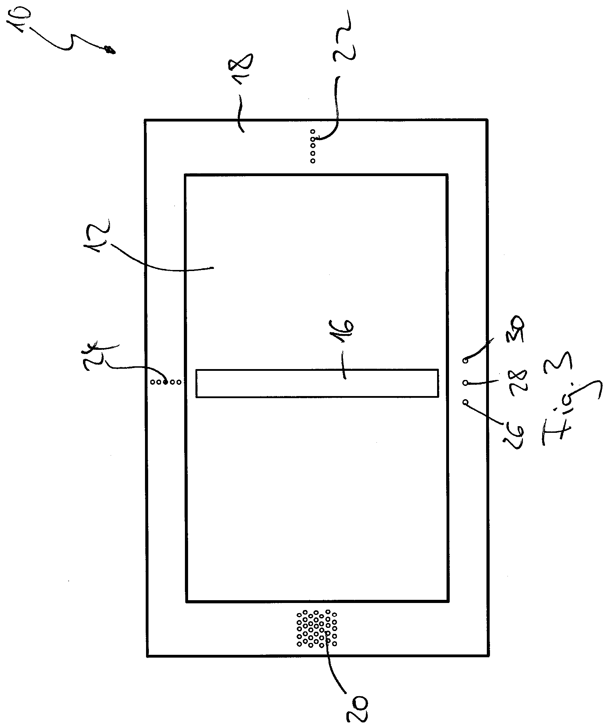

Das Sehzeichengerät 10 umfasst eine Anzeige 12 zum Darbieten von in den Figuren 2 und 3 gezeigten Sehzeichen 14, 16 und einen die Anzeige 12 umrahmenden Rahmen 18. Die Anzeige 12 ist als 3D-Display eines Fernsehers ausgebildet.

Der Rahmen 18 ist zur Befestigung an einem Sehzeichengerät 10 mit einer zentralen Anzeige 12 ausgelegt oder ist Teil des Sehzeichengeräts 10.

Wie in den Figuren deutlich erkennbar ist, umfasst der Rahmen 18 eine Mehrzahl von Lichtquellen 20, 22, 24, 26, 28, 30. Diese Lichtquellen 20, 22, 24, 26, 28, 30 sind als LEDs ausgebildet.

Der Rahmen 18 umfasst eine Lichtquelle 20, die eine Lichtstärke im Bereich von 500 Lumen bis 5000 Lumen aufweist. Diese Lichtstärke hat sich als besonders vorteilhaft erwiesen, um die Blendwirkung von bei Dämmerung oder Nacht entgegenkommenden Fahrzeugen zu simulieren.

Der Rahmen 18 umfasst ferner eine Lichtquelle 22. Die Lichtquelle 22 umfasst mehrere auf einer gedachten Linie angeordnete Einzellichtquellen 23, so dass die Lichtquelle 22 insgesamt zur Aussendung eines linienförmigen horizontalen Lichts ausgelegt ist. Ferner umfasst der Rahmen 18 eine Lichtquelle 24, die mehrere auf einer gedachten Linie angeordnete Einzellichtquellen 25 umfasst, so dass die Lichtquelle 24 insgesamt zur Aussendung eines linienförmigen vertikalen Lichts ausgelegt ist. Die Verwendung einer Lichtquelle 22, 24 im Rahmen, die ein linienförmiges Licht aussendet, ist besonders vorteilhaft, da die beim bekannten Maddox-Test auftretende zentrale Blendung des auf ein Testkreuz blickenden Auges vermieden werden kann und die dann dezentrale Blendung auf eine größere Netzhautfläche verteilt werden kann. Als besonders vorteilhaft hat es sich erwiesen, wenn auf der Anzeige 12 eine horizontale und/oder vertikale Maddox-Testskala dargeboten wird. Zur Durchführung des Maddox-Tests ist das Sehzeichengerät 10 bzw. die Anzeige 12 des Sehzeichengeräts 10 zur Darbietung der in den Figuren 2 und 3 gezeigten Maddox-Testskalen 14, 16 ausgelegt.

Der Rahmen 18 umfasst weiterhin drei jeweils punktförmige Lichtquellen 26, 28, 30, wobei die Lichtquelle 26 als rote Lichtquelle ausgebildet ist, wobei die Lichtquelle 28 als gelbe Lichtquelle ausgebildet ist und wobei die Lichtquelle 30 als grüne Lichtquelle ausgebildet ist. Die Lichtquellen 26, 28, 30 sind linienförmig in einem Abstand von maximal 10 cm zueinander angeordnet. Durch die Verwendung der Lichtquellen 26, 28, 30 in verschiedenen Farben wird die chromatische Aberration des Auges genutzt. Jede Lichtfarbe hat eine andere Refraktion. So kann die Refraktion beim mesopischen oder skotopischen Sehen für die benötigten Farben angepasst werden, bspw. für Verkehrssituationen bei Nacht. Menschen mit einer Fehlsichtigkeit nehmen Zerstreuungskreise der verschiedenen Farben (bspw. grün und rot oder blau und rot) in unterschiedlicher Größe wahr, so dass eine Testperson ihren subjektiven Eindruck entsprechend mitteilen kann. Mit einem derartigen Sehzeichengerät 10 können folglich mit Hilfe der Lichtquellen 26, 28, 30 dämmerungs- und nachtbedingte Effekte bei der Refraktion berücksichtigt werden, so dass bspw. eine für Testpersonen geeignete Nachtfahrbrille bestimmt werden kann.

## Patentansprüche

1. Verfahren zur Bestimmung einer bei Nacht geeigneten Augenkorrektur unter Berücksichtigung dämmerungs- und nachtbedingter Effekte bei der Refraktion an einer Testperson, umfassend folgende Schritte:
- Darbieten der Testperson von Sehzeichen auf einer zentralen Anzeige (12);
- Beleuchten eines Abschnitts eines die Anzeige (12) umgebenden Rahmens (18) durch wenigstens drei am Rahmen linienförmig angeordnete punktförmige Lichtquellen (26, 28, 30) mit Licht von voneinander verschiedener Farben zur Erreichung unterschiedlichen Refraktionen; und
- Bestimmen einer bei Nacht geeigneten Augenkorrektur in Abhängigkeit der Mitteilung des subjektiven Eindrucks der Testperson über die Größe der von der Testperson wahrgenommenen Zerstreuungskreise der verschiedenen Lichtquellen, indem die Refraktion für die verschiedenen Farben angepasst wird.

2. Verfahren nach Anspruch 1, **dadurch gekennzeichnet, dass** der Abschnitt mit Lichtquellen (26, 28, 30) mit Licht von voneinander verschiedenen Farben, vorzugsweise mit einer roten, einer gelben und einer grünen Lichtquelle (26, 28, 30) oder einer roten, einer grünen und einer blauen Lichtquelle (26, 28, 30) beleuchtet wird.

3. Verfahren nach wenigstens einem der vorherigen Ansprüche, **dadurch gekennzeichnet, dass** der Abschnitt mit einer Lichtquelle (20) mit einer Lichtstärke im Bereich von 500 Lumen bis 5000 Lumen beleuchtet wird.

4. Verfahren nach wenigstens einem der vorherigen Ansprüche, **dadurch gekennzeichnet, dass** der Abschnitt mit wenigstens einer dezentralen Lichtquelle (22, 24) dritter Art beleuchtet wird.

5. Verfahren nach wenigstens einem der vorherigen Ansprüche, **dadurch gekennzeichnet, dass** als Lichtquellen (20, 22, 24, 26, 28, 30) LEDs verwendet werden.

6. Verfahren nach wenigstens einem der vorherigen Ansprüche, **dadurch gekennzeichnet, dass** das Verfahren in einem Raum ohne Tageslicht und ohne eine weitere künstliche Lichtquelle durchgeführt wird.

7. Sehzeichengerät (10) zur Durchführung des Verfahrens nach einem der vorhergehenden Ansprüche, umfassend eine zentrale Anzeige (12) zum Darbieten von Sehzeichen (14, 16) und einen Rahmen (18), der zur Umrahmung der Anzeige (12) ausgelegt ist, wobei der Rahmen (18) wenigstens drei linienförmig angeordnete punktförmige Lichtquellen (26, 28, 30) mit Licht von voneinander verschiedenen Farben zur Erreichung der unterschiedlichen Refraktionen umfasst, und wobei die Lichtquellen (26, 28, 30) mit Licht von voneinander verschiedenen Farben in einem Abstand von 0,5 bis 10 cm zueinander angeordnet sind,
wobei die drei punktförmigen Lichtquellen (26, 28, 30) mit Licht von voneinander verschiedenen Farben wenigstens eine rote, eine gelbe und eine grüne Lichtquelle (26, 28, 30) oder wenigstens eine rote, eine grüne und eine blaue Lichtquelle (26, 28, 30) umfassen,
wobei der Rahmen (18) eine weitere Lichtquelle (20) vorsieht, die eine Lichtstärke im Bereich von 500 Lumen bis 5000 Lumen aufweist.

8. Sehzeichengerät (10) nach Anspruch 7, **dadurch gekennzeichnet, dass** wenigstens eine dezentrale Lichtquelle (22, 24) dritter Art vorgesehen ist.

9. Sehzeichengerät (10) nach wenigstens einem der Ansprüche 7 oder 8, **dadurch gekennzeichnet, dass** die Anzeige (12) ein Display eines 3D-Fernsehgeräts ist.

## Claims

1. Method for determining an eye correction that is suitable at night, taking into account twilight and night-time effects of refraction on a test person, comprising the following steps:
- presenting the test person with optotypes on a central display (12);
- illuminating a portion of a frame (18) surrounding the display (12) by at least three point-shaped light sources (26, 28, 30) arranged linearly on the frame, the light sources having light of different colors from each other to achieve different refractions; and
- determining an eye correction that is suitable at night depending on the communication of the subjective impression of the test person regarding the size of the circles of confusion of the different light sources perceived by the test person, by the refraction being adjusted for the different colors.

2. Method according to claim 1, **characterized in that** the portion is illuminated with light sources (26, 28, 30) having light of different colors from each other, preferably with a red, a yellow and a green light source (26, 28, 30) or a red, a green and a blue light source (26, 28, 30).

3. Method according to at least one of the preceding claims, **characterized in that** the portion is illuminated with a light source (20) having a luminous intensity in the range of 500 lumens to 5000 lumens.

4. Method according to at least one of the preceding claims,
**characterized in that** the portion is illuminated with at least one decentralized light source (22, 24) of a third type.

5. Method according to at least one of the preceding claims, **characterized in that** LEDs are used as light sources (20, 22, 24, 26, 28, 30).

6. Method according to at least one of the preceding claims,
**characterized in that** the method is carried out in a room without daylight and without any other artificial light source.

7. Optotype device (10) for carrying out the method according to any of the preceding claims, comprising a central display (12) for presenting optotypes (14, 16) and comprising a frame (18) which is designed to frame the display (12), wherein the frame (18) comprises at least three linearly arranged point-shaped light sources (26, 28, 30) having light of different colors from each other to achieve the different refractions, and wherein the light sources (26, 28, 30) having light of different colors from each other are arranged at a distance of 0.5 to 10 cm from one another,
wherein the three point-shaped light sources (26, 28, 30) having light of different colors from each other comprise at least one red, one yellow and one green light source (26, 28, 30) or at least one red, one green and one blue light source (26, 28, 30),
wherein the frame (18) provides a further light source (20) which has a luminous intensity in the range of 500 lumens to 5000 lumens.

8. Optotype device (10) according to claim 7, **characterized in that** at least one decentralized light source (22, 24) of a third type is provided.

9. Optotype device (10) according to at least one of claims 7 or 8,
**characterized in that** the display (12) is a display of a 3D television.

## Revendications

1. Procédé pour déterminer une correction oculaire appropriée la nuit en tenant compte des effets dus au crépuscule et à la nuit lors de la réfraction sur un sujet, comprenant les étapes suivantes :
- la présentation au sujet d'optotypes sur un affichage (12) central ;
- l'éclairage d'une partie d'un cadre (18) entourant l'affichage (12) par au moins trois sources lumineuses ponctuelles (26, 28, 30) disposées linéairement sur le cadre avec une lumière de couleurs différentes les unes des autres pour obtenir des réfractions différentes ; et
- la détermination d'une correction oculaire appropriée de nuit en fonction de la communication de l'impression subjective du sujet sur la taille des cercles de dispersion des différentes sources lumineuses perçues par le sujet, en adaptant la réfraction pour les différentes couleurs.

2. Procédé selon la revendication 1, **caractérisé en ce que** la partie est éclairée avec des sources lumineuses (26, 28, 30) avec de la lumière de couleurs différentes les unes des autres, de préférence avec une source lumineuse rouge, une source lumineuse jaune et une source lumineuse verte (26, 28, 30) ou une source lumineuse rouge, une source lumineuse verte et une source lumineuse bleue (26, 28, 30).

3. Procédé selon au moins l'une des revendications précédentes, **caractérisé en ce que** la partie est éclairée avec une source lumineuse (20) avec une intensité lumineuse dans la plage de 500 lumens à 5000 lumens.

4. Procédé selon au moins l'une des revendications précédentes, **caractérisé en ce que** la partie est éclairée avec au moins une source lumineuse (22, 24) décentralisée de troisième type.

5. Procédé selon au moins l'une des revendications précédentes, **caractérisé en ce que** des LED sont utilisées comme sources lumineuses (20, 22, 24, 26, 28, 30).

6. Procédé selon au moins l'une des revendications précédentes, **caractérisé en ce que** le procédé est réalisé dans une pièce sans lumière du jour et sans autre source lumineuse artificielle.

7. Appareil à optotypes (10) pour la mise en œuvre du procédé selon l'une quelconque des revendications précédentes, comprenant un affichage (12) central pour présenter des optotypes (14, 16) et un cadre (18) qui est configuré pour encadrer l'affichage (12), dans lequel le cadre (18) comprend au moins trois sources lumineuses ponctuelles (26, 28, 30) disposées linéairement avec de la lumière de couleurs différentes les unes des autres pour obtenir les différentes réfractions, et dans lequel les sources lumineuses (26, 28, 30) avec de la lumière de couleurs différentes les unes des autres sont disposées à une distance de 0,5 à 10 cm les unes des autres,
dans lequel les trois sources lumineuses ponctuelles (26, 28, 30) avec de la lumière de couleurs différentes les unes des autres comprennent au moins une source lumineuse rouge, une source lumineuse jaune et une source lumineuse verte (26, 28, 30) ou au moins une source lumineuse rouge, une source lumineuse verte et une source lumineuse bleue (26, 28, 30),
dans lequel le cadre (18) prévoit une autre source lumineuse (20) qui présente une intensité lumineuse dans la plage de 500 lumens à 5000 lumens.

8. Appareil à optotypes (10) selon la revendication 7, **caractérisé en ce qu'**au moins une source lumineuse (22, 24) décentralisée de troisième type est prévue.

9. Appareil à optotypes (10) selon au moins l'une des revendications 7 ou 8, **caractérisé en ce que** l'affichage (12) est un écran d'un téléviseur 3D.
